# FASCICULE DE BREVET EUROPEEN

(11) **EP 1 097 680 B1**
(45) Date de publication et mention de la délivrance du brevet: **28.09.2005**
(21) Numéro de dépôt: 00420227.1
(22) Date de dépôt: 03.11.2000
(51) Int. Cl.: A61F 2/42

(54) **Prothèse de cheville**
Knöchelprothese
Ankle prosthesis

(30) Priorité: 05.11.1999 FR 9914198
(43) Date de publication de la demande: 09.05.2001
(73) Titulaire: European Foot Platform, 54000 Nancy (FR)
(72) Inventeur: Viladot Perice, Ramon, 08022 Barcelone (ES); Dereymaeker, Greta, 3050 Oud-Heverlee (BE); Diebold, Patrice François, 54000 Nancy (FR); Hintermann, Beat, 4125 Riehen (CH)
(74) Mandataire: Martin, Didier Roland Valéry

(56) Documents cités:
- EP-A- 0 864 305
- DE-U- 8 812 806
- US-A- 4 755 185
- US-A- 5 951 604

## Description

La présente invention se rapporte au domaine technique des prothèses de cheville destinées à permettre le traitement orthopédique de l'articulation de la cheville par implantation d'éléments de prothèse pour restituer l'anatomie de l'articulation de la cheville.

La présente invention concerne une prothèse de cheville comprenant un implant astragalien destiné à être implanté dans ou sur l'astragale, et un élément supérieur comportant un implant tibial destiné à être implanté dans ou sur la base du tibia, ledit élément supérieur et l'implant astragalien étant montés mobiles relativement entre eux par frottement au niveau d'une interface de contact pour permettre les mouvements de la cheville.

Il est déjà connu d'avoir recours à des prothèses de cheville à la suite de chocs traumatiques ayant conduit à un endommagement total ou partiel de l'interligne articulaire de la cheville en vue de restituer au moins en partie l'anatomie articulaire de la cheville.

Ainsi, on connaît déjà des prothèses de cheville comprenant deux implants, à savoir un implant astragalien destiné à être implanté dans ou sur l'astragale, et un implant tibial destiné à être implanté dans ou sur la base du tibia. Ces deux éléments sont implantés dans les os du patient et conformés, par l'intermédiaire de surfaces congruentes, de telle manière que la surface de frottement entre les deux implants soit sensiblement sphérique.

Les prothèses de cheville à deux éléments du type mentionné ci-dessus confèrent à l'articulation de la cheville ainsi recréée une liberté de mouvement satisfaisante. Néanmoins, de telles prothèses ne permettent de reproduire que partiellement l'ensemble des libertés de mouvements de l'articulation naturelle de la cheville puisque la surface de frottement entre les deux implants est très éloignée de la forme physiologique théorique de l'articulation naturelle de la cheville. Ceci étant un système contraint, conduit pour le patient à une limitation de ses capacités de mouvements et induit une élévation du taux de descellement des éléments composant la prothèse.

C'est la raison pour laquelle il a déjà été proposé d'essayer d'améliorer la liberté de mouvements des prothèses de cheville en concevant des prothèses de cheville à trois éléments, à savoir un implant astragalien, un implant tibial et un implant intermédiaire interposé entre l'implant tibial et l'implant astragalien.

L'implant intermédiaire est libre en mouvement entre les deux autres implants et repose sur l'implant tibial. La surface de contact entre l'implant astragalien et l'implant intermédiaire est généralement constituée d'une surface de frottement de forme généralement cylindrique ou sphérique, comme dans le cas des implants à deux éléments.

Une prothèse de cheville à trois éléments procure en conséquence une plus grande liberté de mouvements de l'articulation de la cheville ainsi restituée ce qui procure au patient une possibilité de mouvement se rapprochant de la forme physiologique naturelle de l'articulation de la cheville. Néanmoins, une prothèse de cheville à trois éléments ne reproduit pas de manière complète la forme physiologique naturelle de l'articulation de la cheville. En effet, le déroulement naturel de l'articulation de la cheville implique une attaque latérale du sol avec le pied, suivie par un déroulement du pied impliquant une rotation dirigée progressivement vers l'intérieur du pied. Le déroulement naturel de l'articulation de la cheville n'est en conséquence pas restitué par les prothèses de cheville connues à ce jour.

Par ailleurs, les prothèses de cheville connues à ce jour présentent des moyens de blocage, du genre plots par exemple, solidaires de l'implant tibial pour assurer la solidarisation de l'implant avec l'os tibial. La mise en place de cet implant ne peut s'effectuer par pression puisque le patient est allongé. Ainsi, lors du premier appui au sol, l'implant tibial est comprimé contre l'os tibial ce qui produit un déplacement relatif de l'implant tibial et de l'os tibial. Un tel déplacement provoque des sollicitations mécaniques élevées et incontrôlées au niveau des plots de blocage et de leur logement ce qui induit des déformations, voire des risques de désolidarisation.

Les objets assignés à l'invention visent en conséquence à proposer une nouvelle prothèse de cheville ne présentant pas les inconvénients des prothèses de cheville mentionnées précédemment et reproduisant de la manière la plus fidèle possible l'ensemble des mouvements de déplacement de l'articulation naturelle de la cheville.

Un autre objet de l'invention vise à proposer une nouvelle prothèse de cheville particulièrement adaptée à la reproduction de l'articulation naturelle de la cheville à l'aide d'une prothèse à trois implants.

Un autre objet de l'invention vise à proposer une nouvelle prothèse de cheville permettant une bonne maîtrise des divers mouvements relatifs des implants composant la prothèse.

Un autre objet de l'invention vise à proposer une prothèse de cheville susceptible d'empêcher ou de limiter la création d'ostéophyte gênant le débattement de la cheville.

Un autre objet de l'invention est de proposer une nouvelle prothèse de cheville susceptible d'améliorer les conditions de pose de la prothèse et d'empêcher les déformations incontrôlées des implants une fois posés.

Les objets assignés à l'invention sont atteints à l'aide d'une prothèse de cheville comprenant un implant astragalien destiné à être implanté dans ou sur l'astragale, et un élément supérieur comportant un implant tibial destiné à être implanté dans ou sur la base du tibia, ledit élément supérieur et l'implant astragalien étant montés mobiles relativement entre eux au niveau d'une interface de contact pour permettre les mouvements de la cheville caractérisée en ce que l'interface de contact présente une surface de frottement assimilable à une fraction de surface sensiblement tronconique, ladite surface étant orientée pour que sa partie de plus grand rayon soit tournée sensiblement vers l'extérieur de la cheville.

D'autres objets et avantages de l'invention seront explicités en détail à la lumière de la description qui suit et à l'aide des dessins annexés ci-après, donnés à titre purement illustratifs et non limitatifs, dans lesquels :
- La figure 1 illustre, selon une vue en perspective de trois quart avant, une forme de réalisation d'une prothèse de cheville conforme à l'invention.
- La figure 2 illustre, selon une vue en perspective avant interne, une prothèse de cheville conforme à l'invention.
- La figure 3 illustre, selon une vue de dessus, un exemple de réalisation de l'implant tibial.
- La figure 4 illustre, selon une vue latérale, une variante de réalisation d'un implant astragalien conforme à l'invention.
- La figure 5 illustre, selon une vue schématique en projection, la configuration préférentielle d'un détail de réalisation d'une prothèse conforme à l'invention.
- La figure 6 illustre, selon une vue en perspective, une prothèse conforme à l'invention à deux implants.
- La figure 7 illustre, selon une vue en perspective, une variante de prothèse conforme à l'invention.
- La figure 8 illustre, selon une vue en perspective, une variante de prothèse selon l'invention.

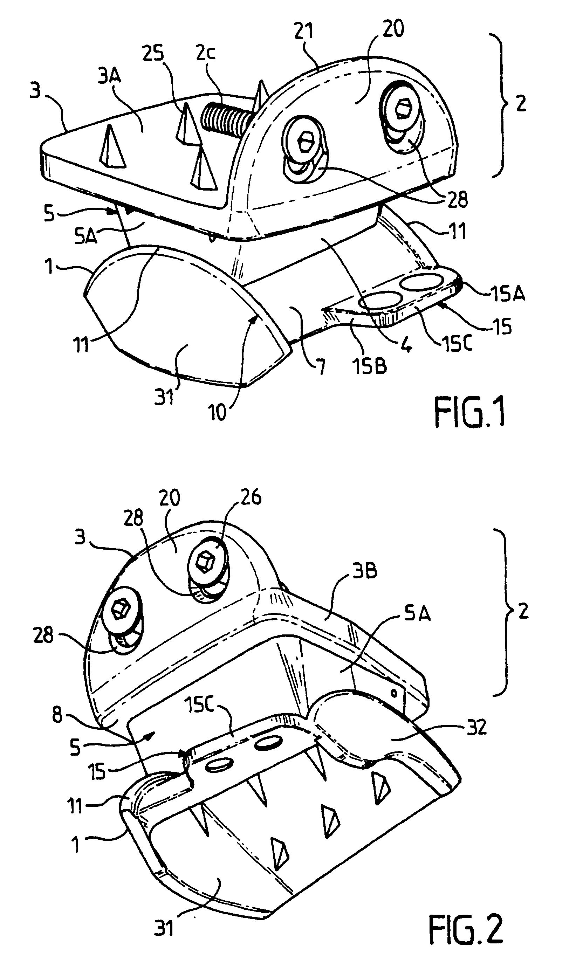

Les figures 1 à 7 montrent une prothèse de cheville conforme à l'invention comprenant un implant astragalien 1 destiné à être implanté dans ou sur l'astragale de l'articulation de la cheville d'un patient. La prothèse de cheville conforme à l'invention comprend également un élément supérieur 2 comportant un implant tibial 3 destiné à être implanté dans ou sur la base du tibia d'un patient.

L'élément supérieur 2 et l'implant astragalien 1 sont montés mobiles relativement entre eux par frottement au niveau d'une interface de contact 4 pour permettre les mouvements de la cheville.

Dans l'exemple de réalisation montré aux figures 1, 2, 7 et 8, l'élément supérieur 2 comporte en outre un implant intermédiaire 5 interposé entre l'implant tibial 3 et l'implant astragalien 1, ledit implant intermédiaire 5 étant en contact par frottement libre avec les deux autres implants par chacune de ses deux faces opposées.

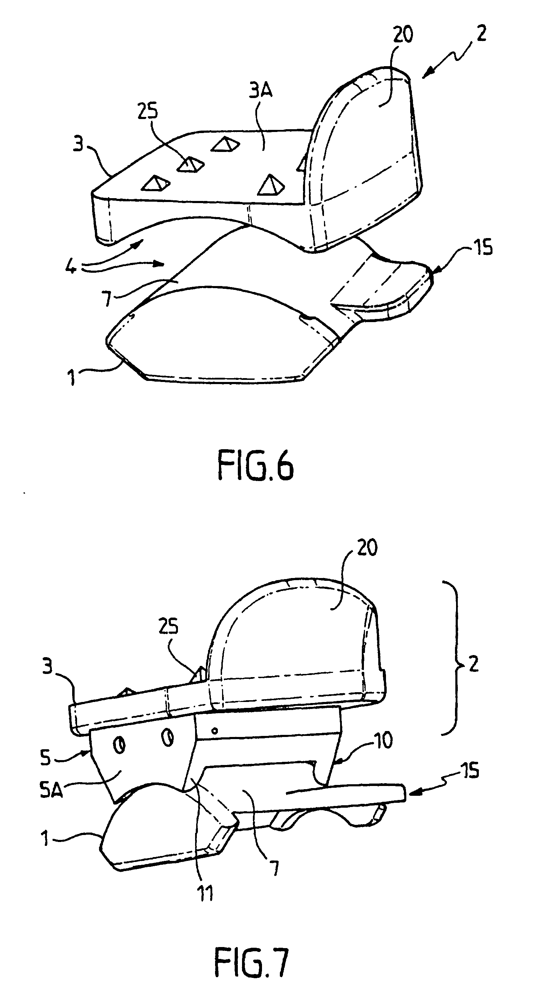

Ainsi, dans la description qui suit, il sera fait constamment référence à une prothèse de cheville comprenant un ensemble de trois implants, à savoir un implant astragalien 1, un implant tibial 3 et un implant intermédiaire 5, étant entendu que la prothèse de cheville selon l'invention ne saurait en aucun cas être limitée à une prothèse de cheville à trois implants. En effet, sans sortir du cadre de l'invention, la prothèse de cheville peut être composée d'un ensemble de deux implants (figure 6), à savoir un implant astragalien 1 et un élément supérieur 2 formé uniquement par un implant tibial 3. Selon cette variante de réalisation, non explicitée en détail dans la description qui suit, l'implant tibial 3 est directement en contact avec l'implant astragalien 1 au niveau de leur interface de contact commune 4.

Ainsi, l'interface de contact entre l'élément supérieur 2 et l'implant astragalien 1 présente une surface de frottement assimilable à une fraction de surface sensiblement tronconique 7 (figure 5), ladite surface étant orientée pour que sa partie de plus grand rayon R soit tournée sensiblement vers l'extérieur de la cheville, c'est-à-dire à l'opposé de l'axe médian du corps, lorsque la prothèse est en place. La surface de frottement est donc définie dans l'espace par une suite de génératrices rectilignes ayant un seul et unique point de convergence pour former la surface sensiblement tronconique. Ceci permet un mouvement complètement libre dans l'espace des différentes pièces de la prothèse, sans faire intervenir d'éventuelles contraintes mécaniques entre les pièces et/ou les tissus biologiques de la cheville.

Dans l'application préférentielle aux prothèses de cheville à trois composants tels que montré aux figures 1 à 4 et 7, 8, l'implant intermédiaire 5 est donc à même de se déplacer par frottement, relativement à l'implant astragalien 1 par une portion de surface tronconique 7 présentant des rayons variables de R à r, ce qui permet d'entraîner le pied sur le côté latéral en flexion dorsale et inversement sur le côté médian en flexion plantaire. Grâce à cette particularité de surface d'articulation, la prothèse de cheville selon l'invention permet de suivre au plus près la forme physiologique naturelle de déplacement de l'articulation de la cheville.

Selon une variante préférentielle de l'invention telle qu'illustrée schématiquement à la figure 5, la valeur de l'angle a au sommet du cône virtuel incorporant la fraction de surface sensiblement tronconique est comprise entre 10° et 35°, le rayon externe R de la partie conique étant compris entre 15 et 30 mm alors que la hauteur h de la zone conique est comprise entre 20 et 50 mm.

L'implant tibial 3 et l'implant astragalien 1 sont avantageusement réalisés en alliage métallique, tel que le chrome cobalt ou en tout autre matériau résistant à l'usure et biocompatible.

Selon les versions préférentielles de l'invention illustrées aux figures 1 à 4, l'implant intermédiaire 5, réalisé par exemple en matière plastique du genre polyéthylène à haute densité ou en céramique, se présente sous la forme d'un polygone irrégulier, avec une surface supérieure plane en contact de glissement avec la face inférieure 8, également plane, de l'implant tibial 3. Selon cette variante préférentielle de réalisation, la surface de frottement relative entre la surface supérieure de l'implant intermédiaire 5 et la face inférieure 8 est donc assimilable à un plan et permet des libertés de mouvements selon ce même plan dans deux directions quelconques de l'espace. Selon cette même variante de réalisation préférentielle, l'implant intermédiaire 5 est en contact de glissement avec la fraction de surface tronconique 7 par une face inférieure présentant une surface de forme conjuguée à la surface tronconique 7, et présentant avantageusement une forme concave et tronconique de petit rayon r et de grand rayon R. La conjugaison parfaite de formes et de dimensions entre la face concave de l'implant intermédiaire 5 et la face convexe de la surface tronconique 7 autorise un débattement libre et sans contrainte de la cheville.

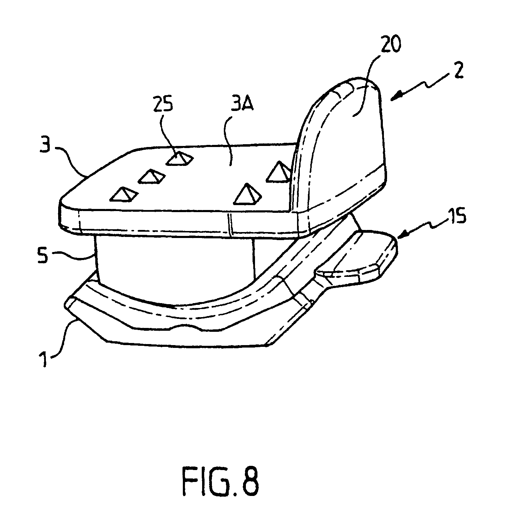

Dans les variantes de réalisations préférentielles montrées aux figures 1 à 4 et 7, la fraction de surface sensiblement tronconique 7 forme la surface de contact externe de l'implant astragalien 1 et est convexe, la face inférieure de l'implant intermédiaire 5 étant alors concave. Selon une variante de réalisation (figure 8), une disposition mécanique inversée pourrait être envisagée sans pour autant sortir du cadre de l'invention, la face inférieure de l'implant intermédiaire 5 étant alors convexe, alors que la surface de contact externe de l'implant astragalien 1 est concave.

Avantageusement, la fraction de surface sensiblement tronconique 7 comporte sur sa surface externe des moyens de guidage 10 du déplacement de l'implant intermédiaire 5 s'étendant selon une direction sensiblement normale à l'axe de symétrie S de ladite surface 7. De cette façon, l'implant intermédiaire 5 et l'implant tibial 3 sont contraints de suivre dans l'espace un déplacement sensiblement normal aux génératrices du tronc de cône hypothétique dont est issue la fraction de surface sensiblement tronconique 7.

Avantageusement, les moyens de guidage 10 sont formés par au moins une et de préférence deux nervures 11, parallèles entre elles, s'élevant à partir de la surface sensiblement tronconique 7 et destinées à enserrer les flancs latéraux 5A de l'implant intermédiaire 5.

Grâce à la présence des nervures parallèles 11, les flancs latéraux 5A de l'implant intermédiaire 5 sont guidés par appui contre lesdites nervures 11 lors du déplacement sur la portion de surface de frottement sensiblement tronconique 7.

A titre de variante (figure 7), les moyens de guidage 10 peuvent être formés par deux nervures 11 s'étendant dans des plans parallèles et issues des flancs latéraux 5A de l'implant intermédiaire 5, lesdites nervures venant en appui et se guidant dans deux rainures correspondantes ménagées dans la masse de la surface sensiblement tronconique 7.

De manière avantageuse, la prothèse de cheville conforme à l'invention comporte un implant astragalien 1 pourvu d'un bouclier antérieur astragalien 15 destiné à assurer la protection de l'articulation de la cheville en empêchant le développement des cellules osseuses dans la partie antérieure de la cheville. Avantageusement, le bouclier antérieur astragalien 15 est formé par un prolongement sensiblement radial de l'implant astragalien 1 dans sa partie antérieure. Tel que montré en particulier à la figure 1, le bouclier antérieur astragalien 15 s'étend à partir du bord antérieur dudit implant en prolongeant sensiblement radialement la surface de frottement sensiblement tronconique 7, en considération de l'axe de symétrie S du tronc de cône hypothétique considéré.

Avantageusement, pour tenir compte de la dissymétrie naturelle du contour de l'os astragalien que vient coiffer l'implant astragalien 1, le bouclier antérieur astragalien 15 s'étend radialement de manière dissymétrique. A cette fin, tel que montré à la figure 1, la longueur du bord latéral intérieur 15B est supérieure à la longueur du bord latéral extérieur 15A de manière à former un bord frontal 15C oblique.

Avantageusement, l'implant tibial 3 est également pourvu d'un bouclier antérieur tibial 20 pour protéger l'articulation de la cheville contre tout développement de cellules osseuses pouvant se développer de manière inconsidérée dans la zone antérieure de la cheville et susceptible de gêner le libre débattement de l'articulation de la cheville.

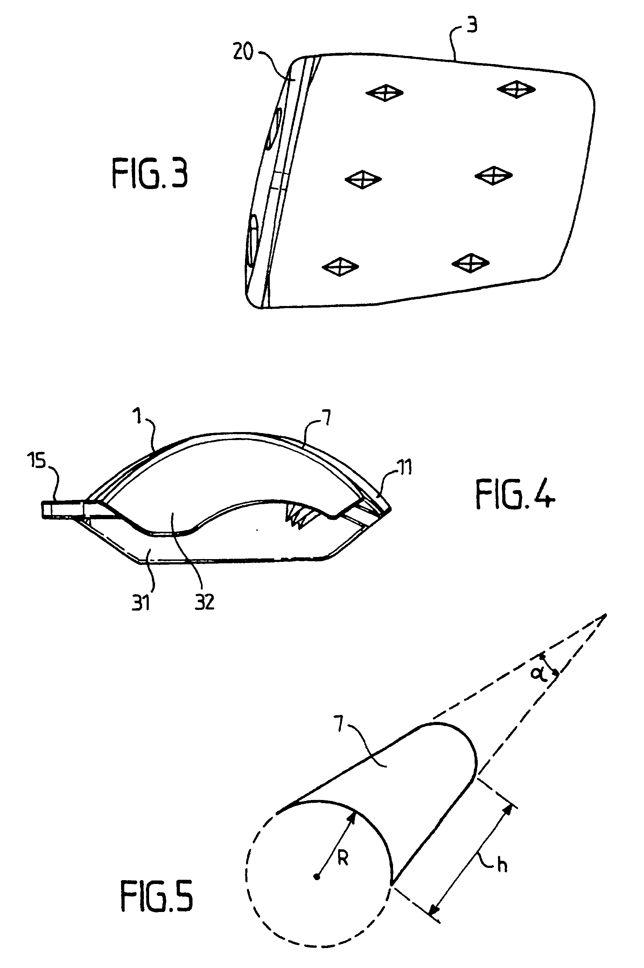

Selon une version particulièrement avantageuse de l'invention, le bouclier antérieur tibial 20 est formé par un prolongement à partir du bord antérieur dudit implant, ledit prolongement s'étendant vers le haut en direction de l'os tibial lorsque ledit implant est en place Ainsi, tel que montré aux figures 1 à 3, le bouclier antérieur tibial 20 s'étend à partir de la face supérieure 3A de l'implant tibial 3, en direction du tibia, selon un plan d'extension quelconque, et par exemple perpendiculaire au plan d'extension de la face 3A. Selon une version particulièrement avantageuse de l'invention (figure 3), le bouclier antérieur tibial 20 épouse la géométrie en forme d'arc sensiblement circulaire de la partie distale du tibia destinée à venir reposer sur la face 3A. A cette fin, le bouclier antérieur tibial 20 présente une forme complexe dissymétrique, le bord antérieur du plateau de l'implant tibial 3 étant oblique et décalé en direction de l'axe médian du patient (figure 3).

Tel qu'illustré à la figure 1, le bouclier antérieur tibial 20 peut présenter une face antérieure sensiblement plane pourvue d'un contour externe sensiblement circulaire.

Avantageusement, l'implant astragalien 1 est limité sur ses deux zones latérales par deux ailes, respectivement externe 31 et interne 32 destinées à former une coiffe avec la partie inférieure concave de la surface sensiblement tronconique 7. De préférence, l'aile externe 31 s'étend sur une hauteur supérieure à celle de l'aile interne 32.

Tel que montré en particulier aux figures 1 à 3, l'implant tibial 3 est destiné à être mis en place par pression contre l'os tibial. Il comporte à cet effet, une surface 3A formant un plateau spécialement préparée, par exemple irrégulière, tel que montré à la figure 3 et obtenue par sablage. A titre de variante, la face 3A peut être pourvue de picots 25 (figure 1) de manière à favoriser un bon accrochage. La mise en place de l'implant tibial 3 est complétée par l'insertion d'une ou plusieurs vis 26 à travers des lumières traversantes appropriées ménagées dans ledit implant tibial pour assurer la fixation de l'os tibial avec ledit implant.

Selon l'invention, le bouclier antérieur tibial 20 est pourvu d'au moins une lumière traversante 28 destinée à assurer le passage d'une vis de blocage 26, ladite lumière étant de dimension supérieure à la section de la vis 26 de manière à permettre un jeu de montage entre l'implant tibial 3 et le tibia. Avantageusement, tel que montré aux figures 1 et 2, la ou les lumières 28 sont de forme oblongue. Grâce à cette conformation, la mise en place de l'implant tibial 3 peut être réalisé en pression sans entraîner une déformation des vis 26. En effet, la mise en place de l'implant tibial 3 s'effectue lorsque le patient est allongé, la mise en compression complète de l'os tibial contre le plateau de l'implant tibial 3 s'effectuant lorsque le patient est debout. Selon l'invention, la possibilité de déplacement relatif des vis 26 dans les lumières 28 oblongues permet un blocage naturel par pression sans endommagement des vis de blocage 26 ou du bouclier antérieur tibial 20 en raison du jeu relatif entre ces deux pièces.

La prothèse de cheville selon l'invention permet en conséquence de reproduire de manière fidèle les possibilités de déplacements relatifs de l'articulation de la cheville naturels grâce à l'existence d'une surface de frottement sensiblement tronconique entre l'implant astragalien 1 et l'implant intermédiaire 5. Par ailleurs, la prothèse de cheville selon l'invention possède une meilleure assise et empêche tout développement de cellules osseuses susceptibles de gêner la libre articulation de la cheville. La mise en place de la prothèse de cheville selon l'invention peut en outre être effectuée sans risque d'endommager en aucune manière l'intégrité des éléments composant la prothèse.

## Revendications

1. Prothèse de cheville comprenant un implant astragalien (1) destiné à être implanté dans ou sur l'astragale et un élément supérieur (2) comportant un implant tibial (3) destiné à être implanté dans ou sur la base du tibia, ledit élément supérieur (2) et l'implant astragalien (1) étant montés mobiles relativement entre eux par frottement au niveau d'une interface de contact (4) pour permettre les mouvements de la cheville, **caractérisée en ce que** l'interface de contact (4) présente une surface de frottement assimilable à une fraction de surface sensiblement tronconique (7), ladite surface étant orientée pour que sa partie de plus grand rayon soit tournée sensiblement vers l'extérieur de la cheville.

2. Prothèse de cheville selon la revendication 1 **caractérisé en ce que** la valeur de l'angle a au sommet du cône virtuel incorporant la fraction de surface sensiblement tronconique est comprise entre 10° et 35°, le rayon externe R de la partie conique étant compris entre 15 et 30 mm alors que la hauteur h de la zone conique est comprise entre 20 et 50 mm.

3. Prothèse de cheville selon l'une des revendications 1 ou 2 **caractérisé en ce que** l'élément supérieur (2) comporte en outre un implant intermédiaire (5) interposé entre l'implant tibial (3) et l'implant astragalien (1), ledit implant intermédiaire (5) étant en contact libre par frottement relatif avec les deux autres implants par chacune de ses deux faces opposées.

4. Prothèse de cheville selon la revendication 3 **caractérisé en ce que** l'implant intermédiaire (5) est en contact de glissement avec l'implant tibial (3) par une surface supérieure plane et en contact de glissement avec la fraction de la surface tronconique (7) par une face inférieure de forme conjuguée.

5. Prothèse de cheville selon l'une des revendications 1 à 4 **caractérisé en ce que** la fraction de surface sensiblement tronconique (7) forme la surface de contact externe de l'implant astragalien (1) et est convexe.

6. Prothèse de cheville selon l'une des revendications 1 à 3 **caractérisé en ce que** la fraction de surface sensiblement tronconique (7) comporte sur sa surface des moyens de guidage (10) du déplacement de l'implant intermédiaire (5), lesdits moyens s'étendant selon une direction sensiblement normale à l'axe de symétrie de ladite surface (7).

7. Prothèse de cheville selon la revendication 6 **caractérisé en ce que** les moyens de guidage (10) sont formés par deux nervures (11) s'élevant à partir de la surface sensiblement tronconique (7).

8. Prothèse de cheville selon l'une des revendications 1 à 7 **caractérisé en ce que** l'implant astragalien (1) est pourvu d'un bouclier antérieur astragalien (15) de protection de l'articulation.

9. Prothèse de cheville selon la revendication 8 **caractérisé en ce que** le bouclier antérieur astragalien (15) est formé par un prolongement sensiblement radial, de l'implant astragalien (1), à partir du bord antérieur dudit implant.

10. Prothèse de cheville selon l'une des revendications 8 ou 9 **caractérisé en ce que** le bouclier antérieur astragalien (15) s'étend radialement de manière dissymétrique de telle façon que son bord latéral intérieur soit d'une longueur supérieure à son bord latéral extérieur.

11. Prothèse de cheville selon l'une des revendications 1 à 10 **caractérisé en ce que** l'implant tibial (3) est pourvu d'un bouclier antérieur tibial (20) pour protéger l'articulation.

12. Prothèse de cheville selon la revendication 11 **caractérisé en ce que** le bouclier antérieur tibial (20) est formé par un prolongement à partir du bord antérieur dudit implant, ledit prolongement s'étendant vers le haut en direction du tibia lorsque ledit implant est en place.

13. Prothèse de cheville selon l'une des revendications 11 ou 12 **caractérisé en ce que** le bouclier antérieur tibial (20) est pourvu d'au moins une lumière traversante (28) destinée à assurer le passage d'une vis de blocage (26) dudit implant, ladite lumière étant de dimension supérieure à la section de la vis (26) de manière à permette un jeu de montage entre l'implant tibial (3) et le tibia.

14. Prothèse de cheville selon la revendication 13 **caractérisé en ce que** la ou les lumières (28) sont de forme oblongue.

## Patentansprüche

1. Knöchelprothese, umfassend ein Sprungbeinimplantat (1), das dazu bestimmt ist, in oder auf dem Sprungbein implantiert zu werden, und ein oberes Element (2), umfassend ein Schienbeinimplantat (3), das dazu bestimmt ist, in oder auf der Schienbeinbasis implantiert zu werden, wobei das obere Element (2) und das Sprungbeinimplantat (1) zueinander durch Reibung im Bereich einer Kontaktfläche (4) beweglich montiert sind, um die Bewegungen des Knöchels zu ermöglichen, **dadurch gekennzeichnet, dass** die Kontaktfläche (4) eine Reibungsfläche aufweist, die mit einem im Wesentlichen kegelstumpfartigen Flächenabschnitt (7) gleichzusetzen ist, wobei die Fläche derart ausgerichtet ist, dass ihr Teil mit dem größten Radius im Wesentlichen zum Äußeren des Knöchels gerichtet ist.

2. Knöchelprothese nach Anspruch 1, **dadurch gekennzeichnet, dass** der Wert des Winkels α an der Spitze des virtuellen Kegels, der den im Wesentlichen kegelstumpfartigen Flächenabschnitt einschließt, zwischen 10° und 35° beträgt, wobei der äußere Radius R des konischen Teils zwischen 15 und 30 mm beträgt, während die Höhe h der konischen Zone zwischen 20 und 50 mm beträgt.

3. Knöchelprothese nach einem der Ansprüche 1 oder 2, **dadurch gekennzeichnet, dass** das obere Element (2) ferner ein Zwischenimplantat (5) umfasst, das zwischen dem Schienbeinimplantat (3) und dem Sprungbeinimplantat (1) angeordnet ist, wobei das Zwischenimplantat (5) durch relative Reibung mit den beiden anderen Implantaten mit jeder ihrer beiden gegenüber liegenden Flächen in Kontakt ist.

4. Knöchelprothese nach Anspruch 3, **dadurch gekennzeichnet, dass** das Zwischenimplantat (5) mit dem Schienbeinimplantat (3) durch eine obere ebene Fläche in Gleitkontakt steht und mit dem kegelstumpfartigen Flächenabschnitt (7) durch eine untere Seite von angepasster Form in Gleitkontakt steht.

5. Knöchelprothese nach einem der Ansprüche 1 bis 4, **dadurch gekennzeichnet, dass** der im Wesentlichen kegelstumpfartige Flächenabschnitt (7) die äußere Kontaktfläche des Sprungbeinimplantats (1) bildet und konvex ist.

6. Knöchelprothese nach einem der Ansprüche 1 bis 3, **dadurch gekennzeichnet, dass** der im Wesentlichen kegelstumpfartige Flächenabschnitt (7) auf seiner Oberfläche Führungsmittel (10) für die Verschiebung der Zwischenimplantat (5) umfasst, wobei sich die Mittel entlang einer im Wesentlichen zur Symmetrieachse der Fläche (7) normalen Richtung erstrecken.

7. Knöchelprothese nach Anspruch 6, **dadurch gekennzeichnet, dass** die Führungsmittel (10) von zwei Rippen (11) gebildet werden, die von der im Wesentlichen kegelstumpfartigen Fläche (7) emporragen.

8. Knöchelprothese nach einem der Ansprüche 1 bis 7, **dadurch gekennzeichnet, dass** das Sprungbeinimplantat (1) mit einem vorderen Sprungbeinschutz (15) zum Schutz des Gelenks versehen ist.

9. Knöchelprothese nach Anspruch 8, **dadurch gekennzeichnet, dass** der vordere Sprungbeinschutz (15) von einer im Wesentlichen radialen Verlängerung des Sprungbeinimplantats (1) ausgehend vom vorderen Rand des Implantats gebildet ist.

10. Knöchelprothese nach einem der Ansprüche 8 oder 9, **dadurch gekennzeichnet, dass** sich der vordere Sprungbeinschutz (15) radial asymmetrisch derart erstreckt, dass sein innerer seitlicher Rand eine größere Länge als sein äußerer seitlicher Rand aufweist.

11. Knöchelprothese nach einem der Ansprüche 1 bis 10, **dadurch gekennzeichnet, dass** das Schienbeinimplantat (3) mit einem vorderen Schienbeinschutz (20) zum Schutz des Gelenks versehen ist.

12. Knöchelprothese nach Anspruch 11, **dadurch gekennzeichnet, dass** der vordere Schienbeinschutz (20) von einer Verlängerung ausgehend vom vorderen Rand des Implantats gebildet ist, wobei sich die Verlängerung nach oben in Richtung des Schienbeins erstreckt, wenn das Implantat eingesetzt ist.

13. Knöchelprothese nach einem der Ansprüche 11 oder 12, **dadurch gekennzeichnet, dass** der vordere Schienbeinschutz (20) mit mindestens einer Queröffnung (28) versehen ist, die dazu bestimmt ist, den Durchgang einer Schraube (26) zur Arretierung des Implantats sicher zu stellen, wobei die Öffnung eine größere Abmessung als der Querschnitt der Schraube (26) aufweist, um ein Montagespiel zwischen dem Schienbeinimplantat (3) und dem Schienbein zu gestatten.

14. Knöchelprothese nach Anspruch 13, **dadurch gekennzeichnet, dass** die Öffnung(en) (28) eine längliche Form besitzt(en).

## Claims

1. Ankle prosthesis comprising a talus implant (1) designed to be implanted in or on the talus, and an upper element (2) having a tibial implant (3) designed to be implanted in or on the base of the tibia, said upper element (2) and the talus implant (1) being mounted so as to be movable relative to each other by friction at a contact interface (4) in order to permit the movements of the ankle, **characterized in that** the contact interface (4) has a friction surface having a shape akin to a fraction of a substantially frustoconical surface (7), said surface being oriented such that its portion of larger radius is directed substantially towards the outside of the ankle.

2. Ankle prosthesis according to Claim 1, **characterized in that** the value of the angle α at the apex of the virtual cone incorporating the fraction of a substantially frustoconical surface is between 10° and 35°, the outer radius R of the conical part being between 15 and 30 mm, while the height h of the conical zone is between 20 and 50 mm.

3. Ankle prosthesis according to one of Claims 1 and 2, **characterized in that** the upper element (2) additionally comprises an intermediate implant (5) interposed between the tibial implant (3) and the talus implant (1), said intermediate implant (5) being in free contact by relative friction with the two other implants via each of its two opposite faces.

4. Ankle prosthesis according to Claim 3, **characterized in that** the intermediate implant (5) is in sliding contact with the tibial implant (3) via a plane upper surface and in sliding contact with the fraction of the frustoconical surface (7) via a lower face of complementary shape.

5. Ankle prosthesis according to one of Claims 1 to 4, **characterized in that** the fraction of substantially frustoconical surface (7) forms the outer contact surface of the talus implant (1) and is convex.

6. Ankle prosthesis according to one of Claims 1 to 3, **characterized in that** the fraction of substantially frustoconical surface (7) includes, on its surface, guide means (10) for guiding the displacement of the intermediate implant (5), said means extending in a direction substantially perpendicular to the axis of symmetry of said surface (7).

7. Ankle prosthesis according to Claim 6, **characterized in that** the guide means (10) are formed by two ribs (11) rising from the substantially frustoconical surface (7).

8. Ankle prosthesis according to one of Claims 1 to 7, **characterized in that** the talus implant (1) is provided with an anterior talus shield (15) for protecting the articulation.

9. Ankle prosthesis according to Claim 8, **characterized in that** the anterior talus shield (15) is formed by a substantially radial extension of the talus implant (1), starting from the anterior edge of said implant.

10. Ankle prosthesis according to one of Claims 8 and 9, **characterized in that** the anterior talus shield (15) extends radially in an asymmetrical manner in such a way that its inner lateral edge is longer than its outer lateral edge.

11. Ankle prosthesis according to one of Claims 1 to 10, **characterized in that** the tibial implant (3) is provided with an anterior tibial shield (20) for protecting the articulation.

12. Ankle prosthesis according to Claim 11, **characterized in that** the anterior tibial shield (20) is formed by an extension starting from the anterior edge of said implant, said extension extending upwards in the direction of the tibia when said implant is in place.

13. Ankle prosthesis according to one of Claims 11 and 12, **characterized in that** the anterior tibial shield (20) is provided with at least one through-hole (28) for the passage of a securing screw (26) for securing said implant, said hole being of a dimension greater than the cross section of the screw (26), in such a way as to permit an assembly clearance between the tibial implant (3) and the tibia.

14. Ankle prosthesis according to Claim 13, **characterized in that** the hole/holes (28) has/have an oblong shape.
